(19) Europäisches Patentamt
European Patent Office
Office européen des brevets

(11) EP 2 934 467 B1

(12) **EUROPEAN PATENT SPECIFICATION**

(45) Date of publication and mention of the grant of the patent:
**26.09.2018 Bulletin 2018/39**

(21) Application number: **12813216.4**

(22) Date of filing: **20.12.2012**

(51) Int Cl.:
***A61K 8/36*** *(2006.01)*
***A61K 8/46*** *(2006.01)*
***A61K 8/49*** *(2006.01)*
***A61Q 11/00*** *(2006.01)*
***A61K 31/4164*** *(2006.01)*

(86) International application number:
**PCT/US2012/070949**

(87) International publication number:
**WO 2014/098867 (26.06.2014 Gazette 2014/26)**

(54) **ORAL CARE COMPOSITION CONTAINING IONIC LIQUIDS**

MUNDPFLEGEZUSAMMENSETZUNG MIT IONISCHEN FLÜSSIGKEITEN

COMPOSITION DE SOIN BUCCAL CONTENANT UN LIQUIDE IONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**28.10.2015 Bulletin 2015/44**

(73) Proprietor: **Colgate-Palmolive Company**
**New York, NY 10022 (US)**

(72) Inventors:
- **PATEL, Madhusudan**
**Somerset, New Jersey 08873 (US)**
- **HASSAN, Mahmoud**
**Somerset, New Jersey 08873 (US)**
- **PAREDES, Rosa**
**North Brunswick, New Jersey 08902 (US)**

(74) Representative: **Wibbelmann, Jobst et al**
**Wuesthoff & Wuesthoff**
**Patentanwälte PartG mbB**
**Schweigerstrasse 2**
**81541 München (DE)**

(56) References cited:
**WO-A1-94/18939**
**WO-A1-2007/144286**
**WO-A1-2012/177277**
**WO-A2-2004/035018**
**WO-A2-2009/125222**
**WO-A2-2011/087621**
**DE-A1-102005 026 355**
**DE-A1-102006 035 830**
**DE-A1-102007 050 767**
**FR-A1- 2 482 859**
**US-A- 3 911 133**
**US-A1- 2005 169 852**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 934 467 B1

## Description

### BACKGROUND OF THE INVENTION

An ionic liquid is a class of salt comprising a cation and an anion that is in liquid at a temperature of 100°C or less and commonly have melting points below room temperature. While not wishing to be bound by theory, ionic liquids generally have much lower symmetry than conventional salts and the charge of cation and anion is distrubuted over a larger volume of the molecule by resonance in ionic liquids which is thought to contribute to their liquid state at much lower temperatures than conventional salts (e.g. NaCl, mp 801°C). Ionic liquids are often composed of a cation comprising a heterocyclic ring and a counter anion, often inorganic in nature. The nature of the cation and anion will determine the hydrophobicity, viscosity, density and other physical parameters and properties of the ionic liquid.

Ionic liquids have been evaluated as environmentally-friendly or 'green' alternatives to conventional organic solvents for a wide range of organic synthetic applications. Ionic liquids have unique characteristics that distinguish them from conventional organic solvents. For example, ionic liquids are non-volatile (i.e. they do not evaporate readily into the atmosphere), they have a high polarity and charge density, they may be hydrophobic or hydrophilic, and they have unique solvating properties. As such, ionic liquids are known to be used in cleaning compositions (for example, as disclosed in US 2006/0090777 A1 and US 7 939 485 B2). A range of ionic liquids are commercially available, or they may be readily synthesized by simple ion-exchange reactions.

A biofilm is a structured group of microorganisms encapsulated within a self-developed polymeric extracellular matrix. Biofilms are typically adhered to a living or inert surface. In the human or animal body biofilms can form on any internal or external surface. Biofilms have been found to be involved in a wide variety of microbial infections in the body and cause a number of conditions including urinary tract infections, middle-ear infections, and in particular, diseases of the oral cavity.

Dental plaque is formed from a biofilm precursor, and is present to some degree on virtually all dental surfaces whether in the oral cavity or on dental instruments used by dentists and hygenists. It comprises a dense microbial layer consisting of a mass of microorganisms embedded in a polysaccharide matrix. Plaque may form on any part of the tooth surface, and is found particularly at the gingival margin, and in cracks in the enamel. The danger associated with the formation of plaque on the teeth lies in the tendency of plaque to build up and eventually produce gingivitis, periodontitis and other types of periodontal disease, as well as dental caries and dental calculus.

Plaque itself adheres very firmly to dental surfaces and rapidly reforms on the tooth surface after it is removed. Current plaque removal methods rely primarily on the mechanical removal of plaque. These methods, which include brushing, brushing with an abrasive toothpaste, flossing, using interdental cleaners, scraping, using sonic energy (e.g. Sonicare toothbrushes) and ultrasound (e.g. Ultreo toothbrushes), in part, rely on a good brushing or flossing technique which many consumers simply do not possess. Moreover, these methods are particularly inefficient in removing stubborn plaque, or plaque hidden deep within cavities and fissures of teeth, between teeth, or within gum pockets.

It is also known in the art to incorporate antimicrobial agents in oral compositions which destroy or retard the growth of bacteria. However, bacteria present in a biofilm or plaque deposit exhibit increased resistance to antimicrobial agents because the dense extracellular matrix and the outer layer of cells protect the bacteria found in the interior of the deposit from the effects of the antimicrobial agents.

There is therefore the need to provide improved methods and compositions for removing plaque which mitigate some of the inefficiencies resulting from a poor brushing/flossing technique and which effectively remove plaque hidden between teeth, within cavities and fissures of teeth, and in gum pockets.

WO 2007/144286 A1 and WO 2009/125222 A2 disclose anti-microbial systems including inter alia certain imidazolium ionic liquids and non-ionic or anionic surfactants.

DE 10 2005 026 355 A1 concerns oral care compositions and discloses compositions for oral and dental hygiene comprising at least one ionic liquid in an acceptable carrier.

### BRIEF SUMMARY OF THE INVENTION

The present invention aims at least partially to meet these needs in the art.

In a first aspect, the present invention provides an oral care composition comprising an ionic liquid, wherein the ionic liquid comprises:

a) an imidazolium cation and

b) an anion selected from the group consisting of acetate, octyl sulfate, or tosylate, wherein the cation is an imidazolium ion having the formula of claim 1, wherein $R^1$ and $R^3$ are independently selected from $C_{1-10}$ alkyl and $C_{2-10}$ alkenyl, and wherein $R^2$ is independently selected from, H, $C_{1-10}$ alkyl and $C_{2-10}$ alkenyl, wherein the ionic liquid is present in the oral care composition at a concentration of 5 mM to 300 mM, and wherein the oral care composition

is provided as a mouth rinse, a toothpaste, oral beads or strips, an irrigation fluid, a plaque removal liquid, a tongue spray, a dental floss, candy, a lozenge, chewing gum, patches or lollipop, wherein the composition comprises a humectant.

Still further optionally, the $C_{1-10}$ alkyl and $C_{2-10}$ alkenyl are linear.

Optionally, $R^1$ is $C_{6-10}$ alkyl or $C_{6-10}$ alkenyl. Further optionally, $R^3$ is methyl.

Typically, $R^2$ is H.

Optionally, $R^1$ is selected from ethyl, butyl, hexyl, decyl, and allyl.

Optionally, the ionic liquid is selected from the group consisting of 1-ethyl-3-methylimidazolium (EMIM) acetate, EMIM tosylate, 1-butyl-3-methylimidazolium (BMIM) octyl sulfate and BMIM acetate. Typically, the oral care composition comprises an abrasive in an amount of less than 0.1 wt% by total weight of the composition. Optionally, the oral care composition comprises an abrasive in an amount of less than 0.01 wt% by total weight of the composition. Further optionally, the oral care composition is substantially free of any abrasives.

Optionally, the ionic liquid is present in the oral care composition at a concentration of about 15 mM to about 250 mM or about 1 mM to about 50 mM.

The oral care composition comprises an orally acceptable carrier for a mouth rinse, toothpaste, oral beads or strips, irrigation fluid, plaque removal liquid, tongue spray, dental floss, candy, lozenge, chewing gum, patches (e.g. intra oral patch similar to smokeless tobacco pouches) and lollipop.

Typically, the oral care composition further comprises one or more agents selected from diluents, bicarbonate salts, pH modifying agents, surfactants, foam modulators, thickening agents, viscosity modifiers, sweeteners, flavorants, pigments, anticaries agents, anticalculus or tartar control agents, abrasives and mixtures thereof.

Optionally, the oral care composition is for removing or reducing plaque, for teeth whitening, and/or for inhibiting the growth of bacteria.

Optionally, the oral care composition is for preventing or treating tooth decay, periodontal disease, gingivitis, or xerostomia (dry mouth).

In a second aspect, the oral care compositions are useful in a method of removing or reducing plaque from the oral cavity of a subject comprising administering a therapeutically effective amount of a composition comprising at least one ionic liquid to the subject.

Optionally, the composition disrupts, detaches and/or dissolves plaque from the oral cavity.

Further optionally, the method comprises preventing or treating tooth decay, periodontal disease or gingivitis.

In a third aspect, the present invention provides the use of the inventive oral care composition for whitening teeth in a subject comprising administering an effective amount of the composition according to the invention.

In a further aspect, the present invention provides an oral care composition useful in a method of reducing the amount of bacteria in the oral cavity of a subject comprising administering a therapeutically effective amount of a composition according to the invention to the subject.

In a fifth aspect, the present invention oral care composition according to a present invention is useful for removing or reducing plaque in the oral cavity of a subject.

## DESCRIPTION OF THE INVENTION

It should be understood that the detailed description, and specific examples, while indicating embodiments of the invention, are intended for purposes of illustration only and are not intended to limit the scope of the invention.

As used throughout, ranges are used as shorthand for describing each and every value that is within the range. Any value within the range can be selected as the terminus of the range.

As used herein, the words "preferred" and "preferably" refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As used herein, the term "about," when applied to the value for a parameter of a composition or method of this invention, indicates that the calculation or the measurement of the value allows some slight imprecision without having a substantial effect on the chemical or physical attributes of the composition or method. If, for some reason, the imprecision provided by "about" is not otherwise understood in the art with this ordinary meaning, then "about" as used herein indicates a possible variation of up to 5% in the value.

As referred to herein, all compositional percentages are by weight of the total composition, unless otherwise specified.

In some embodiments, the present invention provides an oral care composition comprising an ionic liquid, wherein the ionic liquid comprises:

a) an imidazolium cation, and

b) an anion selected from the group consisting of salicylate, acetate, halide, phosphate, alkyl phosphate, sulfate, alkyl sulfate, and tosylate.

## Imidazolium

An imidazolium cation in the context of this patent has the formula represented by the structure below, as defined in the claims.

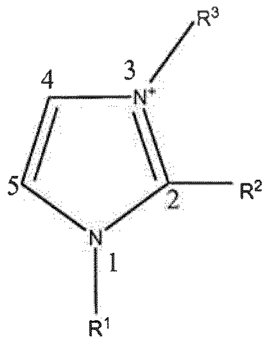

As used herein, the term "alkyl" refers to a saturated aliphatic hydrocarbon, including straight-chain, branched-chain and cycloalkyl groups of 1 to 20 carbon atoms. Alkyl groups include methyl, ethyl, propyl, butyl, pentyl, hexyl, heptyl, octyl, nonyl and decyl.

As used herein, the term "cycloalkyl" refers to a $C_{3-8}$ cyclic hydrocarbon.

As used herein, the term "alkenyl" refers to an unsaturated, open chain hydrocarbon with 2 to 20 carbon atoms and with one or more carbon-carbon double bonds. For example, alkenyl groups include allyl and vinyl.

$R^1$, $R^2$ and $R^3$ may be the same or different. The alkyl or alkenyl groups mentioned herein may be linear or branched. Typically, the alkyl or alkenyl groups are linear.

In a preferred embodiment, $R^1$ and $R^3$ are independently selected from $C_{1-10}$ alkyl and $C_{2-10}$ alkenyl, and $R^2$ is independently selected from, H, $C_{1-10}$ alkyl and $C_{2-10}$ alkenyl.

In some embodiments, the $C_{1-10}$ alkyl and $C_{2-10}$ alkenyl are linear. In other embodiments, the $C_{1-10}$ alkyl and $C_{2-10}$ alkenyl are branched.

In some embodiments, $R^1$ is $C_{4-10}$ alkyl or $C_{4-10}$ alkenyl. In other embodiments, $R^1$ is alkyl or alkenyl. In yet further embodiments, $R^1$ is $C_{8-10}$ alkyl or $C_{8-10}$ alkenyl.

In a preferred embodiment, $R^3$ is methyl.

Typically, $R^2$ is H. Optionally, $R^1$ is selected from $C_{1-10}$ alkyl, or from $C_{4-10}$ alkyl, or from alkyl, $R^2$ is H, and $R^3$ is methyl. Alternatively, $R^1$ is selected from $C_{2-10}$ alkenyl, or from $C_{4-10}$ alkenyl, or from $C_{6-10}$ alkenyl, $R^2$ is H, and $R^3$ is optionally methyl.

In some embodiments, $R^1$, $R^2$, and $R^3$ are independently selected from H, $C_{4-10}$ alkyl and $C_{4-10}$ alkenyl, or from H, alkyl and alkenyl, or from H, $C_{8-10}$ alkyl and $C_{8-10}$ alkenyl.

In a preferred embodiment, $R^1$ is selected from $C_{4-10}$ alkyl and $C_{4-10}$ alkenyl, or from alkyl and $C_{6-10}$ alkenyl, or from $C_{8-10}$ alkyl and $C_{8-10}$ alkenyl, $R^2$ is H, and $R^3$ is optionally methyl.

In one embodiment, $R^1$, $R^2$ and $R^3$ are methyl.

In a typical embodiment, $R^1$ is a methyl, ethyl, propyl, butyl, or pentyl, $R^2$ is H, and $R^3$ is methyl.

In another embodiment, $R^1$ is alkyl (hexyl, heptyl, octyl, nonyl or decyl), $R^2$ is H and $R^3$ is methyl.

In yet another embodiment, $R^1$ is allyl, $R^2$ is H and $R^3$ is methyl.

In a further embodiment, $R^1$ is vinyl, $R^2$ is H and $R^3$ is methyl.

In some embodiments, $R^1$ and $R^2$ are independently selected from $C_{1-5}$ alkyl (methyl, ethyl, propyl, butyl, or pentyl), and $R^3$ is methyl.

In other embodiments, $R^1$ and $R^2$ are independently selected from alkyl (hexyl, heptyl, octyl, nonyl or decyl), and $R^3$ is methyl.

In yet further embodiments, $R^1$ is vinyl or allyl, $R^2$ is selected from methyl, ethyl, propyl, butyl, or pentyl, and $R^3$ is methyl.

## Anions

The anion is selected from the group consisting of acetate, octylsultate or tosylate.

In another embodiment, the anion is tosylate.

In a further embodiment, the anion is acetate.

**Ionic liquid**

The term "ionic liquid" used in the context of the present invention means a salt comprising comprising a cation and an anion that is in liquid at a temperature of 100°C or less and commonly have melting points below room temperature.

Any anion mentioned above may be used in combination with any of the imidazolium ions defined above to form the oral care composition of the present invention.

The ionic liquid is selected from the group consisting of 1-ethyl-3-methylimidazolium (EMIM) acetate, EMIM tosylate, 1-butyl-3-methylimidazolium (BMIM) octyl sulfate and BMIM acetate.

The ionic liquid is present in the oral care composition in a concentration of about 5 mM to about 300 mM or from about 10mM to about 270 mM

Preferably, the ionic liquid is present in the oral care composition in a concentration of about 150 mM to about 250 mM, or from about 180 mM to about 220 mM or about 1 mM to about 50 mM.

In some embodiments, the oral care composition of the invention does not contain any other antibacterial or whitening agent.

**Abrasives**

Whilst, the compositions of the present invention may optionally further comprise an abrasive which may be useful, for example, as a polishing agent, it has been found that oral care compositions comprising ionic liquids as defined herein, are effective in removing biofilm or plaque, and whitening teeth, without the need for substantial amounts of abrasives. This is advantageous because abrasives can damage enamel and expose dentine tissues with repeated use, particularly, in subjects with soft enamel caused by disease or excessive exposure to food acids.

In one embodiment, the oral care composition comprises an abrasive in an amount of less than 0.1 wt % by total weight of the composition.

In another embodiment, the oral care composition comprises an abrasive in an amount of less than 0.01 wt % by total weight of the composition.

In yet another embodiment, the composition is substantially free, or free, of any abrasives.

Suitable optional abrasives include silica, for example in the form of precipitated silica or as admixed with alumina, insoluble phosphates, calcium carbonate, and mixtures thereof. Among insoluble phosphates useful as abrasives are orthophosphates, polymetaphosphates and pyrophosphates. Illustrative examples are dicalcium orthophosphate dihydrate, calcium pyrophosphate, calcium pyrophosphate, tricalcium phosphate, calcium polymetaphosphate and insoluble sodium polymetaphosphate.

**Carrier**

Among useful carriers for optional inclusion in a composition of the invention are diluents, bicarbonate salts, pH modifying agents, surfactants, foam modulators, thickening agents, viscosity modifiers, humectants, sweeteners, flavorants, pigments, anticaries agents, and anticalculus or tartar control agents or abrasives. Carriers should be selected for compatibility with each other and with other ingredients of the composition.

Water is a preferred diluent and in some compositions such as mouthwashes, water is commonly accompanied by an alcohol, e.g., ethanol. The weight ratio of water to alcohol in a mouthwash composition is generally 1:1 to 20:1, for example 3:1 to 20:1 or 4:1 to 10:1. In a whitening liquid, the weight ratio of water to alcohol can be within or below the above ranges, for example, 1:10 to 2:1.

In a further embodiment, the composition of the invention comprises at least one bicarbonate salt, useful for example to impart a "clean feel" to teeth and gums due to effervescence and release of carbon dioxide. Any orally acceptable bicarbonate can be used, including without limitation, alkali metal bicarbonates such as sodium and potassium bicarbonates, ammonium bicarbonate and the like. One or more bicarbonate salts are optionally present in a total amount of about 0.1 wt % to about 50 wt %, for example about 1 wt % to 20 wt %, by total weight of the composition.

In a still further embodiment, the composition of the invention comprises at least one pH modifying agent. Such agents include acidifying agents to lower pH, basifying agents to raise pH, and buffering agents to control pH within a desired range. For example, one or more compounds selected from acidifying, basifying and buffering agents can be included to provide a pH of 2 to 10, or in various illustrative embodiments, 2 to 8, 3 to 9, 4 to 8, 5 to 7, 6 to 10, 7 to 9, etc. Any orally acceptable pH modifying agent can be used, including without limitation, carboxylic, phosphoric and sulfonic acids, acid salts (*e.g.,* monosodium citrate, disodium citrate, monosodium malate, *etc*.), alkali metal hydroxides such as sodium hydroxide, carbonates such as sodium carbonate, bicarbonates, sesquicarbonates, borates, silicates,

phosphates (*e.g.*, monosodium phosphate, trisodium phosphate, pyrophosphate salts, *etc.*), imidazole and the like. One or more pH modifying agents are optionally present in a total amount effective to maintain the composition in an orally acceptable pH range.

In a still further embodiment, the composition of the invention comprises at least one surfactant. Any orally acceptable surfactant, most of which are anionic, nonionic or amphoteric, can be used. Suitable anionic surfactants include without limitation, water-soluble salts of $C_{8-20}$ alkyl sulfates, sulfonated monoglycerides of $C_{8-20}$ fatty acids, sarcosinates, taurates and the like. Illustrative examples of these and other classes include sodium lauryl sulfate, sodium coconut monoglyceride sulfonate, sodium lauryl sarcosinate, sodium lauryl isoethionate, sodium laureth carboxylate and sodium dodecyl benzenesulfonate. Suitable nonionic surfactants include without limitation, poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation, derivatives of $C_{8-20}$ aliphatic secondary and tertiary amines having an anionic group such as carboxylate, sulfate, sulfonate, phosphate or phosphonate. A suitable example is cocoamidopropyl betaine. One or more surfactants are optionally present in a total amount of about 0.01 wt% to about 10 wt %, for example, from about 0.05 wt % to about 5 wt %, or from about 0.1 wt % to about 2 wt % by total weight of the composition.

In a still further embodiment, the composition of the invention comprises at least one foam modulator, useful for example to increase amount, thickness or stability of foam generated by the composition upon agitation. Any orally acceptable foam modulator can be used, including without limitation, polyethylene glycols (PEGs), also known as polyoxyethylenes. High molecular weight PEGs are suitable, including those having an average molecular weight of 200,000 to 7,000,000, for example 500,000 to 5,000,000, or 1,000,000 to 2,500,000. One or more PEGs are optionally present in a total amount of about 0.1 wt % to about 10 wt %, for example from about 0.2 wt % to about 5 wt %, or from about 0.25 wt % to about 2 wt%, by total weight of the composition.

In a still further embodiment, the composition of the invention comprises at least one thickening agent, useful for example to impart a desired consistency and/or mouth feel to the composition. Any orally acceptable thickening agent can be used, including without limitation, carbomers, also known as carboxyvinyl polymers, carrageenans, also known as Irish moss and more particularly t-carrageenan (iota-carrageenan), cellulosic polymers such as hydroxyethylcellulose, carboxymethylcellulose (CMC) and salts thereof, e.g., CMC sodium, natural gums such as karaya, xanthan, gum arabic and tragacanth, colloidal magnesium aluminum silicate, colloidal silica and the like. A preferred class of thickening or gelling agents includes a class of homopolymers of acrylic acid crosslinked with an alkyl ether of pentaerythritol or an alkyl ether of sucrose, or carbomers. Carbomers are commercially available from B. F. Goodrich as the Carbopol® series. Particularly preferred Carbopols include Carbopol 934, 940, 941, 956, 974P, and mixtures thereof. One or more thickening agents are optionally present in a total amount of from about 0.01 wt % to 15 wt%, for example from about 0.1 wt% to about 10 wt%, or from about 0.2 wt % to about 5 wt%, by total weight of the composition.

In a still further embodiment, the composition of the invention comprises at least one viscosity modifier, useful for example to inhibit settling or separation of ingredients or to promote re-dispersibility upon agitation of a liquid composition. Any orally acceptable viscosity modifier can be used, including without limitation, mineral oil, petrolatum, clays and organomodified clays, silica and the like. One or more viscosity modifiers are optionally present in a total amount of from about 0.01 wt % to about 10 wt %, for example, from about 0.1 wt% to about 5 wt%, by total weight of the composition.

In a still further embodiment, the composition of the invention comprises at least one humectant. Any orally acceptable humectant can be used, including without limitation, polyhydric alcohols such as glycerin, sorbitol, xylitol or low molecular weight PEGs. Most humectants also function as sweeteners. One or more humectants are optionally present in a total amount of from about 1 wt% to about 70 wt%, for example, from about 1 wt% to about 50 wt%, from about 2 wt% to about 25 wt%, or from about 5 wt% to about 15 wt%, by total weight of the composition.

In a still further embodiment, a composition of the invention comprises at least one sweetener, useful for example to enhance taste of the composition. Any orally acceptable natural or artificial sweetener can be used, including without limitation dextrose, sucrose, maltose, dextrin, dried invert sugar, mannose, xylose, ribose, fructose, levulose, galactose, corn syrup (including high fructose corn syrup and corn syrup solids), partially hydrolyzed starch, hydrogenated starch hydrolysate, sorbitol, mannitol, xylitol, maltitol, isomalt, aspartame, neotame, saccharin and salts thereof, dipeptide-based intense sweeteners, cyclamates and the like. One or more sweeteners are optionally present in a total amount depending strongly on the particular sweetener(s) selected, but typically 0.005 wt% to 5 wt%, by total weight of the composition.

In a still further embodiment, a composition of the invention comprises at least one flavorant, useful for example to enhance taste of the composition. Any orally acceptable natural or synthetic flavorant can be used, including without limitation vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, *etc.,* bean- and nut-derived flavors such as coffee, cocoa, cola, peanut, almond, *etc.,* adsorbed and encapsulated flavorants and the like. Also encompassed

within flavorants herein are ingredients that provide fragrance and/or other sensory effect in the mouth, including cooling or warming effects. Such ingredients illustratively include menthol, menthyl acetate, menthyl lactate, camphor, eucalyptus oil, eucalyptol, anethole, eugenol, cassia, oxanone, α-irisone, propenyl guaiethol, thymol, linalool, benzaldehyde, cinnamaldehyde, N-ethyl-p-menthan-3 -carboxamine, N,2,3 -trimethyl-2-isopropylbutanamide, 3-(1-menthoxy)-propane-1,2-diol, cinnamaldehyde glycerol acetal (CGA), menthone glycerol acetal (MGA) and the like. One or more flavorants are optionally present in a total amount of from about 0.01 wt % to about 5 wt %, for example, from about 0.1 wt % to about 2.5wt %, by total weight of the composition.

In a still further embodiment, a composition of the invention may comprise at least one colorant. Colorants herein include pigments, dyes, lakes and agents imparting a particular luster or reflectivity such as pearling agents. Any orally acceptable colorant can be used, including without limitation talc, mica, magnesium carbonate, calcium carbonate, magnesium silicate, magnesium aluminum silicate, silica, titanium dioxide, zinc oxide, red, yellow, brown and black iron oxides, ferric ammonium ferrocyanide, manganese violet, ultramarine, titaniated mica, bismuth oxychloride and the like. One or more colorants are optionally present in a total amount of from about 0.001 wt% to about 20 wt%, for example, from about 0.01 wt% to about 10 wt %, or from about 0.1 wt % to about 5 wt%, by total weight of the composition.

In some embodiments, the composition comprises a fluoride ion source. Fluoride ion sources include, but are not limited to: stannous fluoride, sodium fluoride, potassium fluoride, potassium monofluorophosphate, sodium monofluorophosphate, ammonium monofluorophosphate, sodium fluorosilicate, ammonium fluorosilicate, amine fluoride such as olaflur (N'-octadecyltrimethylendiamine-N,N,N'-tris(2-ethanol)-dihydrofluoride), ammonium fluoride, and combinations thereof. In certain embodiments the fluoride ion source includes stannous fluoride, sodium fluoride, amine fluorides, sodium monofluorophosphate, as well as mixtures thereof. In certain embodiments, the oral care composition of the invention may also contain a source of fluoride ions or fluorine-providing ingredient in amounts sufficient to supply about 50 to about 5000 ppm fluoride ion, e.g., from about 100 to about 1000, from about 200 to about 500, or about 250 ppm fluoride ion. Fluoride ion sources may be added to the compositions of the invention at a level of about 0.001 wt % to about 10 wt %, e.g., from about 0.003 wt % to about 5 wt %, 0.01 wt % to about 1 wt, or about 0.05 wt %. However, it is to be understood that the weights of fluoride salts to provide the appropriate level of fluoride ion will obviously vary based on the weight of the counter ion in the salt, and one of skill in the art may readily determine such amounts. A preferred fluoride salt may be sodium fluoride.

The composition of the present invention optionally comprises a saliva stimulating agent useful, for example, in amelioration of dry mouth. Any orally acceptable saliva stimulating agent can be used, including without limitation food acids such as citric, lactic, malic, succinic, ascorbic, adipic, fumaric and tartaric acids, and mixtures thereof. One or more saliva stimulating agents are optionally present in saliva stimulating effective total amount.

The composition of the present invention optionally incorporates one or more antisensitivity agents, e.g., potassium salts such as potassium nitrate, potassium bicarbonate, potassium chloride, potassium citrate, and potassium oxalate; capsaicin; eugenol; strontium salts; zinc salts; chloride salts and combinations thereof. Such agents may be added in effective amounts, e.g., from about 1 wt % to about 20 wt % by weight based on the total weight of the composition, depending on the agent chosen. The compositions of the present invention may also be used to treat hypersensitivity by blocking dentin tubules when applied to a tooth.

In some embodiments, the composition of the invention further comprises an antioxidant. Any orally acceptable antioxidant can be used, including butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, and mixtures thereof.

In another embodiment, the composition comprises an orally acceptable zinc ion source useful, for example, as an antimicrobial, anticalculus or breath-freshening agent. One or more such sources can be present. Suitable zinc ion sources include without limitation zinc acetate, zinc citrate, zinc gluconate, zinc glycinate, zinc oxide, zinc sulfate, sodium zinc citrate and the like. One or more zinc ion sources are optionally and illustratively present in a total amount of from about 0.05 wt% to about 3 wt%, for example from about 0.1 wt % to about 1 wt%, by total weight of the composition.

The composition of the present invention may additionally optionally comprise a tartar control (anticalculus) agent as provided below. Tartar control agents among those useful herein include salts of the specified agents, including alkali metal and ammonium salts. The agents include: phosphates and polyphosphates (for example pyrophosphates), polyaminopropanesulfonic acid (AMPS), polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and. Useful inorganic phosphate and polyphosphate salts include monobasic, dibasic and tribasic sodium phosphates, sodium tripolyphosphate, tetrapolyphosphate, mono-, di-, tri- and tetrasodium pyrophosphates, sodium trimetaphosphate, sodium hexametaphosphate and mixtures thereof. Other useful tartar control agents include polycarboxylate polymers and polyvinyl methyl ether/maleic anhydride (PVM/MA) copolymers, such as GANTREZ®.

In some embodiments, the composition of the present invention further comprises a nutrient. Suitable nutrients include vitamins, minerals, amino acids, and mixtures thereof. Vitamins include Vitamins C and D, thiamine, riboflavin, calcium pantothenate, niacin, folic acid, nicotinamide, pyridoxine, cyanocobalamin, para-aminobenzoic acid, bioflavonoids, and mixtures thereof. Nutritional supplements include amino acids (such as L-tryptophan, L-lysine, methionine, threonine, levocarnitine and L-carnitine), lipotropics (such as choline, inositol, betaine, and linoleic acid), and mixtures thereof.

Delivery

The oral care composition of the present invention preferably comprises an orally acceptable carrier for use in a product such as a mouth rinse (including dual phase mouthwash), toothpaste, actives in beads/strips, irrigation fluids, plaque removal fluids, bead formulas, formulations to be delivered through devices such as pens, back of a toothbrush and front of a toothbrush, formulations to be delivered through porous wicking materials, interdental brushes, fluid encased dental strips, floss impregnated or coated with the formulations or dried formulations, portables, oral trays, hard or soft candy, lozenge with a soft plaque dissolving liquid inside, lollipops with the plaque dissolving formulation imbedded into the lickable "candy" that can also help control tongue bacteria, peelable gels, patches, formulations for pop-rocks that upon popping, spread a fine mist of the formulation around oral cavity, tongue cleaners with plaque dissolving strips and dental strips. Accordingly, opportunities exist for professional use of the compositions of the present invention (e.g. during cleanings, irrigations, or aggressive periodontal procedures, such as root planning & scaling). The composition of the invention may be provided in any of the products defined herein.
In one embodiment, the composition of the invention can be dried into powder and utilized in a portable sachet. For example, upon mixing such a powder with a suitable solvent such as water, a rinse may be created to remove plaque, proteins and other debris in the mouth.
In another embodiment, the composition of the invention can be dried with abrasives such as silica, calcium carbonate or soft capsules that upon addition of small amount of water, creates a paste to brush away the plaque.
Formulations that increase the substantivity of ionic liquids onto a surface could be expected to increase the efficacy of biofilm, and hence plaque removal. For example, Tween 20 while also functioning as a surfactant, is also a wetting agent. Therefore, incorporation of such an agent could be expected to increase the wettability and spreading of a mouth rinse formulation according to the present invention, over the soft and hard tissue, increasing the formulation’s propensity for plaque dissolution and removal.

**Methods of Use**

The composition according to the present invention may be administered to or applied to a human or other animal subject. The composition may be suitable for administration or application to the oral cavity of a human or animal subject. Typically, the composition is for reducing or removing dental plaque. The reduction or removal of plaque may occur through an inhibition of biofilm (a plaque precursor) formation and/or degradation of microbial biofilm.
The present invention further provides a composition as defined above for preventing or treating a disease condition of the oral cavity. Typically, the disease condition is caused by plaque. The disease condition may be selected from tooth decay, periodontal disease, gingivitis or xerostomia (dry mouth).
Accordingly, the present invention provides a composition as defined above for use as a medicament.
The present invention further provides a use of an oral care composition according to the invention for whitening teeth in a subject comprising administering an effective amount of the composition.
The invention is further illustrated in the following non-limiting examples. (Oral care compositions according to the invention are marked with an asterisk)

Examples

Example 1 - Dissolution of plaque

Dental plaque samples were obtained from subjects and treated with water to remove any water soluble actives. The samples were centrifuged at 13,000 g and the supernatant was collected and discarded. The remaining plaque that was insoluble in water was used to evaluate the plaque solubilizing potential of the ionic liquids.
Ionic liquids were added to the plaque sample, and the plaque sample was vortex mixed at room temperature. To ensure that the plaque was not simply suspended in the ionic liquid, the plaque containing vial with the ionic liquid was subsequently centrifuged at 13,000 g. Any undissolved plaque was visible as a pellet, and in this way, plaque dissolution could be visually monitored.
Plaque may form a suspension when mixed with water and that, on centrifugation, it is visible as an insoluble pellet. On addition of 1-ethyl-3-methylimidazolium bromide, the size of the plaque pellet is reduced, indicating that 1-ethyl-3-methylimidazolium bromide is effective in dissolving plaque. Similar results are obtained with choline acetate, choline salicylate and 1, 2, 4- trimethylpyrazolium methylsulfate.

Example 2 - Protocol for *in vitro* biofilm removal

A 3-day old biofilm provided a sufficiently robust biofilm to help differentiate the efficacy of different prototype formulations in biofilm removal.

The 3-day old biofilm was grown on a 24 well plate using the artificial mouth consortium of bacteria (*A. naeslundii*, *S. oralis*, *V. parvula*, *L. casei, F. nucleatum*) and *S. mutans.* To generate the biofilm, plates were inoculated with saliva overnight to form a pellicle. The consortium of bacteria (1 mL) at an OD of ~ 0.2 was added to each well. The bacterial media (Tryptic Soy Broth with 6 % sucrose) was changed after 48 hours. The 3-day old biofilm was treated with the prototype formulation (500 μL) for 15 minutes on a plate shaker at 300 rpm. After incubation, the supernatant was discarded. The optical density of each well was measured and the percentage reduction in optical density relative to the control (water) was calculated to determine the effect of the prototype formulation on biofilm removal.

As described herein, dental plaque is formed from a biofilm precursor. Therefore, the 3-day old biofilm serves as a good model to investigate the effects of test compounds on plaque removal.

Example 3 - Impact of chain length on biofilm removal

Using the protocol of Example 2, the effects of ionic liquids on biofilm reduction were tested. The percentage removal of a 3-day old biofilm using 0.28M concentration of different ionic liquids, formulated in the base prototype mouth rinse formation of Table 1. Water, untreated biofilm, and the commercially available mouthwash formulations with either CPC (cetyl pyridinium chloride) or sodium monofluorophosphate acted as negative controls for the experiment.

Table 1

| Ingredient Name | Percentage |
|---|---|
| Demineralized water | 38.63 |
| Propylene Glycol | 10.00 |
| Sorbitol non-browning / non-crys NF-sol | 20.00 |
| 99.0%-101.0% Vegetable Glycerin | 20.00 |
| Surfactant - Tween 20 | 1.125 |
| Flavor K91-6525 | 0.12 |
| CPC | 0.075 |
| Sucralose | 0.05 |
| Ionic Liquids | 10.00 |
| Total | 100.00 |

The ionic liquids tested were as follows:

1-ethyl-3-methylimidazolium (EMIM) chloride (Cl), EMIM bromide (Br), EMIM ethyl sulfate, EMIM diethyl phosphate, EMIM acetate (OAc), EMIM tosylate, 1-butyl-3-methylimidazolium (BMIM) chloride (Cl), BMIM bromide (Br), BMIM methyl sulfate, BMIM octyl sulfate, BMIM acetate (OAc), 1-allyl-3-methylimidazolium (AMIM) chloride, 1-decyl-3-methylimidazolium (DMIM) chloride, 1,2,3-trimethylimidazolium methyl sulfate, 1,2,3-trimethylpyrazolium (MMMPZ) methyl sulfate, and MTEOA methyl sulfate (tris(2-hydroxyethyl)methylammonium sulfate).

Table 2 indicates the percentage removal of a 3-day old biofilm using 0.28M concentration of different ionic liquids, formulated in the base prototype mouth rinse formation of Table 1, as compared to control compositions water, commercially available mouthwash (MW) formulations with either CPC (cetyl pyridinium chloride) or five enzymes to combat biofilm, and untreated biofilm.

Table 2

| Sample Name | Percent Removal of a 3-day Oral Biofilm |
|---|---|
| Untreated | -1.3 |
| DI water | -3.7 |
| MW with CPC (0.075%) | 13.4 |

(continued)

| Sample Name | Percent Removal of a 3-day Oral Biofilm |
|---|---|
| MW with five enzymes | 30.0 |
| EMIM Cl | 44.3 |
| BMIM Cl | 43.8 |
| AMIM Cl | 44.8 |
| DMIM Cl | 57.3 |
| EMIM Br | 48.6 |
| BMIM Br | 48.0 |
| BMIM MeSO4 | 53.9 |
| TMIM MeSO4 | 51.8 |
| MTEOA MeSO4 | 52.8 |
| MMMPz MeSO4 | 57.8 |
| EMIM Ethyl SO4 | 48.1 |
| BMIM Octyl SO4 * | 58.6 |
| EMIM diethyl PO4 | 52.7 |
| BMIM OAc * | 55.9 |
| EMIMOAc * | 52.1 |
| EMIM Tosylate * | 59.5 |

As seen in Table 2, each of the formulated ionic liquids were effective in removing oral biofilm by at least 40% or more. In particular, ionic compounds comprising an imidazolium cation, and an anion selected from salicylate, acetate, halide, alkyl phosphate, alkyl sulfate, and tosylate, are significantly more effective than the commercially available or control compositions in reducing biofilm formation. These compounds remove biofilm by an amount that is 2 to 6 times more than the amount removed by the commercially available mouth rinses.

Additionally, it can be seen from Table 2 that biofilm removal increases as the length of the side chain on the imidazolium cation (at the 1-position) is increased. In particular, DMIM chloride is significantly more effective in removing biofilm than EMIM chloride and BMIM chloride.

Furthermore, it can be seen from Table 2 that when the counterion is changed from chloride to bromide for EMIM and BMIM, there is an increase in biofilm removal by at least ~4%.

Thus, it may be concluded that ionic liquids having a longer chain in their core cation moiety and having a larger halide ion such as a bromide ion, enables greater biofilm removal.

Table 2 also reveal that in general, methyl sulfate anions perform better than the halide ions. Additionally, when the alkyl chain of the sulfate ion is increased, (for example, biofilm removal for BMIM methyl sulfate and BMIM octyl sulfate may be compared), biofilm removal is markedly enhanced.

Therefore, in conclusion, compositions comprising ionic liquids, in particular, wherein the ionic liquids comprise an imidazolium ion, are very effective in removing 3-day old biofilm, which serves as a model for removing plaque.

Example 4

Using the protocol of Example 2, the effects of varying the anion type in EMIM- and BMIM-based ionic liquids, on removal of a 3-day old biofilm, were investigated.

Table 3 illustrates the percentage removal of a 3-day old biofilm using 0.28M concentration of different EMIM-based ionic liquids having different anions, formulated in the base prototype mouth rinse formation of Table 1.

Table 3

| EMIM Cationic Series | |
|---|---|
| **Anion** | **Percent Biofilm Removed** |
| Cl | 44.3 |
| Ethylsulfate | 48.1 |
| Br | 48.6 |
| Acetate * | 52.1 |
| Diethyl phosphate | 52.7 |
| Tosylate * | 59.5 |

Table 4 illustrates the percentage removal of a 3-day old biofilm using 0.28M concentration of different BMIM-based ionic liquids having different anions, formulated in the base prototype mouth rinse formation of Table 1.

Table 4

| BMIM Cationic Series | |
|---|---|
| **Anion** | **Percent Biofilm Removal** |
| Cl | 43.8 |
| Br | 48.0 |
| Methylsulfate | 53.9 |
| Acetate * | 55.8 |
| Octylsulfate * | 58.6 |

Table 3 illustrates that the anions diethyl phosphate, acetate and tosylate are most effective in removing biofilm. Efficacy of biofilm removal appears to increase with negatively resonance stabilized species such as acetate and tosylate.

Table 4 illustrates that acetate and octylsulfate anions are more effective in removing biofilm than the halide ions.

Table 5 illustrates a direct comparison between EMIM and BMIM, with different anions, in removing biofilm.

Table 5

| | **Percent Biofilm Removed** | |
|---|---|---|
| | **EMIM** | **BMIM** |
| **Chloride** | 44.3 | 43.8 |
| **Bromide** | 48.6 | 48.0 |
| **Acetate** * | 52.1 | 55.8 |

It can clearly be seen that acetate anions are most effective in removing biofilm, followed by bromide ions and chloride ions, respectively.

Example 5 - Biofilm removal by other imidazolium compounds

Using the protocol of Example 2, the effects of 1-allyl-3-methylimidazolium (AMIM) chloride, 1,2,3-trimethylimidazolium (TMIM) methyl sulfate, and 1-decyl-3-methylimidazolium (DMIM) chloride on removal of a 3-day old biofilm, were compared. The results are illustrated in Table 6.

Table 6

| Other Ionic Liquids | Percent Biofilm Removed |
|---|---|
| AMIM Chloride | 44.8 |
| TMIM Methylsulfate | 51.8 |
| MTEOA Methylsulfate | 52.8 |
| DMIM Chloride | 57.3 |
| MMMPz Methylsulfate | 57.9 |

It can clearly be seen from Table 6 that DMIM chloride is most effective in removing biofilm. It can also be concluded that imidazolium cations with unsaturated side chains (e.g. AMIM chloride) are effective in removing biofilm.

Example 6 - Teeth whitening effect of imidazolium-based ionic liquids

Mouthwash formulations of 1-ethyl-3-methylimidazolium (EMIM) with the anions tosylate, bromide, chloride and ethylsulfate, were evaluated for *in-vitro* removal of tooth stains.

The method of determining the effects of imidazolium-based ionic liquids on teeth whitening was carried out as follows:

The initial L*a*b* measurements of dried artificially stained human teeth were captured. The teeth were then soaked in 1 ml of sample solution for one hour, and sample was replenished at 30 minutes. After treatment, teeth were soaked in DI water for approximately 10 minutes. The teeth were left to dry at least overnight, and final L*a*b* measurements were then taken. (L*a*b* refers to stain score in accordance with the Commission International de L'Eclairage Laboratory (CIELAB) color scale. L* (lightness-darkness scale), a* (red-green chroma) and b* (yellow-blue chroma)).

The whitening efficacy was determined as follows:

$$\Delta W^* = W^*final - W^*initial$$

where

$$W^* = (a^*2 + b^*2 + (L^*-100)2)½$$

All the EMIM-based compounds were evaluated at 1M concentration in a prototype mouthwash formulation containing hydrogen peroxide, as illustrated in Table 7 (Prototype mouth wash formulation containing EMIM-based ionic liquids at 1M concentration.)

Table 7

| Prototype Whitening MW Base | |
|---|---|
| **Ingredient Name** | **Weight %** |
| Glycerin | 0.000 |
| Propylene Glycol | 2.500 |
| Sorbitol | 10.000 |
| Surfactant-Tween 20 | 1.500 |
| Ionic Liquid (1M) | 30.000 |
| Sodium Benzoate | 0.110 |
| CPC | 0.075 |
| Sucralose | 0.050 |
| Citrate Buffer | 10.000 |
| Flavor (K91-5916) | 0.150 |

(continued)

| Prototype Whitening MW Base | |
|---|---|
| Ingredient Name | Weight % |
| Water | 45.615 |
| Total | 100.000 |

Table 8 illustrates the change in tooth whiteness effected by various EMIM-based liquids. A mouthwash (MW) with 1.5% hydrogen peroxide ($H_2O_2$) was used as a peroxide-containing control for the experiment. It can be seen that the tooth whitening capacities of the imidazolium-based ionic liquids decrease in the order: EMIM tosylate, EMIM Br, and EMIM Cl/EMIM ethyl sulfate. (EMIM Cl and EMIM ethyl sulfate produced similar whitening effects). Changes in teeth whiteness (W), lightness (L) and shade (E) were also assessed.

Table 8

| Sample Name | ΔW | ΔE | ΔL |
|---|---|---|---|
| EMIM EtSO4 | -2.99 | 4.41 | 3.40 |
| EMIM Br | -7.98 | 9.75 | 8.86 |
| EMIM Cl | -3.40 | 4.88 | 3.86 |
| EMIM Tos * | -13.90 | 15.53 | 15.03 |
| MW with 1.5% $H_2O_2$ | -3.73 | 4.05 | 3.96 |

Again, EMIM-tosylate, a non-peroxide, provided the most significant tooth whitening benefit through dissolution and removal of surface tooth stains.

Example 7 - antibacterial activity of imidazolium-based ionic liquids

The effects of 1-decyl-3-methylimidazolium (DMIM) chloride on bacterial growth were investigated *in vitro.* DMIM chloride was incorporated into a prototype mouth rinse as illustrated in Table 9. Growth inhibition of *A. viscosus* was assessed when using DMIM chloride in an amount of 0.01 wt%, 0.1 wt% and 1 wt% in the prototype mouth rinse. The growth inhibition was measured as a change in optical density over time. Table 9 depicts the formulation of 1-decyl-3-methylimidazolium chloride as a mouth rinse.

Table 9

| Ingredient Name | Wt % Plaque dissolving formula |
|---|---|
| Propylene Glycol | 4.00 |
| Sorbitol non-browning / non-crys NF-sol | 7.00 |
| 99.0%-101.0% Vegetable Glycerin | 7.00 |
| Flavor K91-6525 | 0.15 |
| Polyoxyl 40 hydrated castor oil NF | 1.00 |
| Sucralose | 0.001 |
| 1-decyl-3-methylimidazolium chloride | 0.001-10% |
| Demineralized water | q.s |
| Total | 100.00 |

When formulated at 1 wt% concentration, 1-decyl-3-methylimidazolium chloride provides greater growth inhibition of *A. viscosus* than a mouthwash containing cetylpyridinium chloride. Growth inhibition of *A. viscosus* is maintained even when 1-decyl-3-methylimidazolium chloride is formulated at 0.312 wt % concentration.

Example 8 - short interval kill test (SIKT)

SIKT determines the kill effect of a test article at a predetermined exposure time. Briefly, a culture of *A. viscosus* was incubated with 1-decyl-3-methylimidazolium, formulated in the prototype mouth rinse according to Table 9. The reaction was neutralized after 30 seconds by adding a neutralizing broth. The reaction mixture was further diluted and plated on MCA (Microbial Count Agar) plates for viable bacterial count.

When 1-decyl-3-methylimidazolium chloride is formulated in the prototype mouth rinse according to Table 9 at 0.3, 0.6 and 1 wt% concentration, it is able to kill *A. viscosus* within 30 seconds.

Example 9 - minimum inhibitory concentration of 1-decyl-3-methylimidazolium chloride.

In order to determine the minimum concentration of 1-decyl-3-methylimidazolium chloride required to inhibit the growth of *A. viscosus,* an *A. viscosus* culture was incubated for 24 hours with varying concentrations of 1-decyl-3-methylimidazolium chloride, and bacterial growth inhibition was measured by taking optical density readings at 610 nm.

The minimum inhibitory concentration of 1-decyl-3-methylimidazolium chloride required to inhibit the growth of *A. viscosus* is 125 ppm.

Example 10 - formulations of 1-decyl-3-methylimidazolium chloride in oral care delivery vehicles.

Table 10 shows a typical formulation of 1-decyl-3-methylimidazolium chloride in a bead formulation.

Table 10

| Ingredient Name | Wt % Plaque dissolving formula |
|---|---|
| Castor Oil | 43.5 |
| Flavor | 15 |
| WS-3 -Cooling sensate | 1.5 |
| 10% Sucralose sln | 5 |
| Ionic Liquid | 0.3-5 |
| Propylene glycol | q.s |
| Total | 100.00 |

Table 11 shows a typical formulation of 1-decyl-3-methylimidazolium chloride in a wick delivery device or in an interdental wicking brush

Table 11

| Ingredient Name | Wt % Plaque dissolving formula |
|---|---|
| Glycerin | 15 |
| Propylene glycol | 30 |
| Flavor (89-186) | 8 |
| WS3-Cooling sensate | 3 |
| 10% Sucralose soln | 5 |
| Ionic Liquid | 0.3-10 |
| Water pH adjusted if necessary | q.s |
| Total | 100.00 |

Example 11 - formulation of ionic liquid in a chewing gum

Xylitol, gum base, isomalt, gum arabic, flavors, maltitol syrup, titanium dioxide, coloring, shellac, carnauba wax, BHT (butylhydroxytoluene; to preserve freshness), and ionic liquid.

**Claims**

1. An oral care composition comprising an ionic liquid and an orally acceptable carrier, wherein the ionic liquid comprises:

   a) an imidazolium cation and
   b) an anion selected from the group consisting of acetate, octyl sulfate, or tosylate, wherein the cation is an imidazolium ion having the formula,

   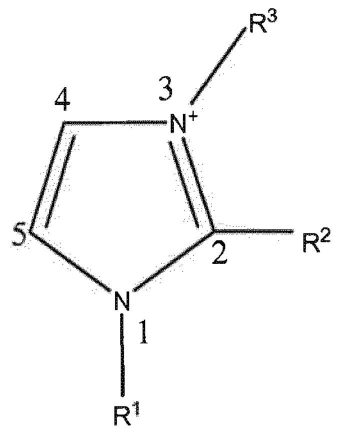

   wherein $R^1$ and $R^3$ are independently selected from $C_{1-10}$ alkyl and $C_{2-10}$ alkenyl, and wherein $R^2$ is independently selected from, H, $C_{1-10}$ alkyl and $C_{2-10}$ alkenyl, wherein the ionic liquid is present in the oral care composition at a concentration of 5 mM to 300 mM, and wherein the oral care composition is provided as a mouth rinse, a toothpaste, oral beads or strips, an irrigation fluid, a plaque removal liquid, a tongue spray, a dental floss, candy, a lozenge, chewing gum, patches or lollipop, wherein the composition comprises a humectant.

2. The oral care composition of claim 1, wherein $R^1$ is $C_{6-10}$ alkyl or $C_{6-10}$ alkenyl.

3. The oral care composition of claim 1, wherein $R^2$ is H.

4. The oral care composition of any of claims 1 to 3, wherein $R^3$ is methyl.

5. The oral care composition any of claims 1 to 4, wherein the ionic liquid is selected from the group consisting of 1-ethyl-3-methylimidazolium (EMiM) acetate, EMIM tosylate, 1-butyl-3-methyimidazolium (BMiM) octyl sulfate and BMIM acetate.

6. The oral care composition of any preceding claim, wherein the oral care composition comprises an abrasive in an amount of less than 0.1 wt% by total weight of the composition, optionally wherein the oral care composition comprises an abrasive in an amount of less than 0.01 wt% by total weight of the composition, further optionally wherein the composition is substantially free of any abrasives.

7. The oral care composition of any preceding claim, wherein the ionic liquid is present in the oral care composition in an amount of 0.1 wt% to 30 wt% based on the total weight of the composition, optionally in an amount of 0.5 wt% to 20 wt% based on the total weight of the composition, further optionally in an amount of 5 wt% to 15 wt% based on the total weight of the composition or in an amount of 8 wt% to 10 wt% based on the total weight of the composition.

8. The oral care composition of any preceding claim, wherein the ionic liquid is present in the oral care composition at a concentration of 15 mM to 250 mM.

9. The oral care composition of any preceding claim, wherein the composition further comprises one or more agents selected from diluents, bicarbonate salts, pH modifying agents, surfactants, foam modulators, thickening agents, viscosity modifiers, sweeteners, flavorants, pigments, anticaries agents, anticalculus or tartar control agents, abrasives and mixtures thereof.

10. The oral care composition according to any of claims 1 to 9, for use in removing or reducing plaque in the oral cavity

of a subject, or for use in reducing the amount of bacteria in the oral cavity of a subject.

**11.** Use of an oral care composition according to any of claims 1 to 9 for whitening teeth in the oral cavity of a subject.

## Patentansprüche

**1.** Mundpflegezusammensetzung umfassend eine ionische Flüssigkeit und einen oral annehmbaren Träger, worin die ionische Flüssigkeit umfasst:

a) ein Imidazolium Kation und
b) ein Anion ausgewählt aus einer Gruppe bestehend aus Azetat, Octylsulfat, oder Tosylat, wobei das Kation ein Imidazolium Ion ist mit der Formel,

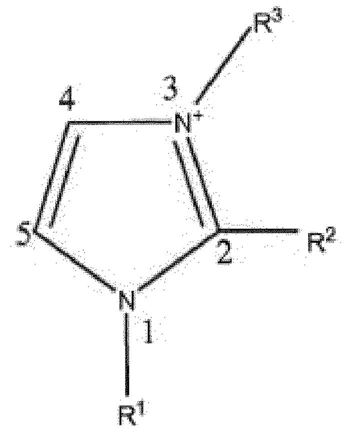

worin $R^1$ und $R^3$ unabhängig voneinander ausgewählt sind aus $C_{1-10}$-Alkyl und $C_{2-10}$-Alkenyl und worin $R^2$ unabhängig ausgewählt ist aus H, $C_{1-10}$-Alkyl und $C_{2-10}$-Alkenyl, worin die ionische Flüssigkeit in der Mundpflegezusammensetzung bei einer Konzentration von 5 mM bis 300 mM vorliegt und wobei die Mundpflegezusammensetzung vorgelegt wird als eine Mundspülung, eine Zahnpasta, orale Perlen oder Streifen, eine Spülflüssigkeit, eine Plaqueentfernungsflüssigkeit, ein Zungenspray, Zahnseide, Süßigkeit, Lutschtablette, Kaugummi, Flicken oder Lutscher, wobei die Zusammensetzung ein Befeuchtungsmittel umfasst.

**2.** Mundpflegezusammensetzung nach Anspruch 1, worin $R^1$ $C_{6-10}$-Alkyl oder $C_{6-10}$-Alkenyl ist.

**3.** Mundpflegezusammensetzung nach Anspruch 1, wobei $R^2$ H ist.

**4.** Mundpflegezusammensetzung nach irgendeinem der Ansprüche 1 bis 3, worin $R^3$ Methyl ist.

**5.** Mundpflegezusammensetzung nach irgendeinem der Ansprüche 1 bis 4, worin die ionische Flüssigkeit ausgewählt ist aus der Gruppe bestehend aus 1-Ethyl-3-Methylimidazolium (EMiM) Acetat, EMIM Tosylat, 1-Butyl-3-Methyimidazolium (BMiM) Octylsulfat und BMIM Acetat.

**6.** Mundpflegezusammensetzung nach irgendeinem vorhergehenden Anspruch, worin die Mundpflegezusammensetzung ein Abrasivstoff umfasst in einer Menge von weniger als 0.1 Gew.% auf das Gesamtgewicht der Zusammensetzung, worin die Mundpflegezusammensetzung gegebenenfalls ein Abrasivstoff in einer Menge von weniger als 0.01 Gew.% auf das Gesamtgewicht der Zusammensetzung umfasst, weiterhin worin die Zusammensetzung gegebenenfalls frei von irgendwelchen Abrasivstoffen ist.

**7.** Mundpflegezusammensetzung nach irgendeinem vorhergehenden Anspruch, worin die ionische Flüssigkeit in der Mundpflegezusammensetzung in einer Menge von 0.1 Gew.% bis 30 Gew.%, bezogen auf das Gesamtgewicht der Zusammensetzung vorliegt, gegebenenfalls in einer Menge von 0.5 Gew.% bis 20 Gew.%, bezogen auf das Gesamtgewicht der Zusammensetzung, weiterhin gegebenenfalls in einer Menge von 5 Gew.% bis 15 Gew.%, bezogen auf das Gesamtgewicht der Zusammensetzung, oder einer Menge von 8 Gew.% bis 10 Gew.%, bezogen auf das Gesamtgewicht der Zusammensetzung.

8. Mundpflegezusammensetzung nach irgendeinem vorhergehenden Anspruch, worin die ionische Flüssigkeit in der Mundpflegezusammensetzung bei einer Konzentration von 15 mM bis 250 mM vorliegt.

9. Mundpflegezusammensetzung nach irgendeinem vorhergehenden Anspruch, worin die Zusammensetzung weiterhin ein oder mehrere Agenzien umfasst ausgewählt aus Verdünnungsmittel, Bikarbonat Salzen, pH-Modifiziermitteln, Tensiden, Schaummodulatoren, Verdickungsmitteln, Viskositätsmodifizierer, Süßstoffen, Geschmacksstoffen, Farbstoffen, Antikariesmittel, Antizahnstein- oder Zahnbelagkontrollmittel, Abrasivstoffe und Mischungen davon.

10. Mundpflegezusammensetzung nach irgendeinem der Ansprüche 1 bis 9 zur Verwendung bei der Entfernung oder Verminderung von Plaque in der Mundhöhle eines Subjektes, oder zur Verwendung in der Reduzierung von Bakterienmenge in der Mundhöhle eines Subjektes.

11. Verwendung einer Mundpflegezusammensetzung gemäß irgendeinem der Ansprüche 1 bis 9 zur Aufhellung von Zähnen in der Mundhöhle eines Subjektes.

## Revendications

1. Composition de soin buccal comprenant un liquide ionique et un support acceptable par voie orale, dans laquelle le liquide ionique comprend :

   a) un cation imidazolium et
   b) un anion choisi dans le groupe constitué par l'acétate, l'octyl sulfate ou le tosylate,
   dans laquelle le cation est un ion imidazolium ayant la formule,

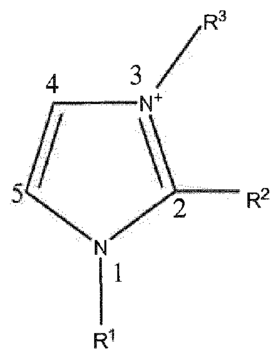

   dans laquelle $R^1$ et $R^3$ sont indépendamment choisis parmi un alkyle en $C_{1-10}$ et un alcényle en $C_{2-10}$, et dans laquelle $R^2$ est indépendamment choisi parmi, un H, un alkyle en $C_{1-10}$ et un alcényle en $C_{2-10}$, dans laquelle le liquide ionique est présent dans la composition de soin buccal à une concentration de 5 mM à 300 mM, et dans laquelle la composition de soin buccal est fournie sous forme d'un rince-bouche, d'une pâte dentifrice, de perles ou de bandelettes orales, d'un liquide d'irrigation, d'un liquide d'élimination de la plaque dentaire, d'un vaporisateur pour la langue, d'un fil dentaire, d'un bonbon, d'un losange, d'un chewing-gum, de timbres ou d'une sucette, dans laquelle la composition comprend un humectant.

2. Composition de soin buccal selon la revendication 1, dans laquelle $R^1$ est un alkyle en $C_{6-10}$ ou un alcényle en $C_{6-10}$.

3. Composition de soin buccal selon la revendication 1, dans laquelle $R^2$ est un H.

4. Composition de soin buccal selon l'une quelconque des revendications 1 à 3, dans laquelle $R^3$ est un méthyle.

5. Composition de soin buccal selon l'une quelconque des revendications 1 à 4, dans laquelle le liquide ionique est choisi dans le groupe constitué par l'acétate de 1-éthyl-3-méthylimidazolium (EMiM), le tosylate d'EMIM, l'octyl sulfate de 1-butyl-3-méthylimidazolium (BMiM) et l'acétate de BMIM.

6. Composition de soin buccal selon l'une quelconque des revendications précédentes, dans laquelle la composition de soin buccal comprend un abrasif en une quantité inférieure à 0,1% en poids du poids total de la composition,

éventuellement dans laquelle la composition de soin buccal comprend un abrasif en quantité inférieure à 0,01% en poids du poids total de la composition, en outre éventuellement dans laquelle la composition est sensiblement exempte de tout abrasif.

**7.** Composition de soin buccal selon l'une quelconque des revendications précédentes, dans laquelle le liquide ionique est présent dans la composition de soin buccal en une quantité de 0,1% en poids à 30 % en poids par rapport au poids total de la composition, éventuellement en une quantité de 0,5 % en poids à 20 % en poids par rapport au poids total de la composition, en outre éventuellement en une quantité de 5 % en poids à 15% en poids par rapport au poids total de la composition ou en une quantité de 8 % en poids à 10% en poids par rapport au poids total de la composition.

**8.** Composition de soin buccal selon l'une quelconque des revendications précédentes, dans laquelle le liquide ionique est présent dans la composition de soin buccal à une concentration de 15 mM à 250 mM.

**9.** Composition de soin buccal selon l'une quelconque des revendications précédentes, dans laquelle la composition comprend en outre un ou plusieurs agents choisis parmi les diluants, les sels de bicarbonate, les agents régulateurs de pH, les tensioactifs, les modulateurs de mousse, les agents épaississants, les modificateurs de viscosité, les édulcorants, les aromatisants, les pigments, les agents anticarie, les agents antitartre ou les agents de régulation du tartre, les abrasifs et les mélanges de ceux-ci.

**10.** Composition de soin buccal selon l'une quelconque des revendications 1 à 9, pour une utilisation dans l'élimination ou la réduction de la plaque dentaire dans la cavité buccale d'un sujet, ou pour une utilisation dans la réduction de la quantité de bactéries dans la cavité buccale d'un sujet.

**11.** Utilisation d'une composition de soin buccal selon l'une quelconque des revendications 1 à 9 pour le blanchiment des dents dans la cavité buccale d'un sujet.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20060090777 A1
- US 7939485 B2
- WO 2007144286 A1
- WO 2009125222 A2
- DE 102005026355 A1